(19) Europäisches Patentamt
European Patent Office
Office européen des brevets

(11) Publication number: **0 202 697 B1**

# (12) EUROPEAN PATENT SPECIFICATION

(45) Date of publication of patent specification: **27.12.91**

(51) Int. Cl.5: **C07C 209/86**, C07C 211/45

(21) Application number: **86200683.0**

(22) Date of filing: **22.04.86**

## (54) Separation process for para-fluoro-aniline.

(30) Priority: **21.05.85 NL 8501447**

(43) Date of publication of application:
**26.11.86 Bulletin 86/48**

(45) Publication of the grant of the patent:
**27.12.91 Bulletin 91/52**

(84) Designated Contracting States:
**BE CH DE FR GB IT LI NL SE**

(56) References cited:
**FR-A- 1 135 873**
**US-A- 4 532 352**

(73) Proprietor: **SHELL INTERNATIONALE RESEARCH MAATSCHAPPIJ B.V.**
**Carel van Bylandtlaan 30**
**NL-2596 HR Den Haag(NL)**

(72) Inventor: **Prillwitz, Peter Ernst**
**Badhuisweg 3**
**NL-1031 CM Amsterdam(NL)**
Inventor: **Wevers, Jan Hendrik**
**Badhuisweg 3**
**NL-1031 CM Amsterdam(NL)**
Inventor: **Gilkerson, Terence**
**Badhuisweg 3**
**NL-1031 CM Amsterdam(NL)**

(74) Representative: **Tuijn, Jan Warnaar et al**
**Shell Internationale Research Maatschappij B.V., Patents, Licensing & Trade Marks Division, P.O. Box 302**
**NL-2501 CH The Hague(NL)**

EP 0 202 697 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The invention relates to a process for separating a para-fluoroaniline from a corresponding para-unsubstituted aniline.

Para-fluoroanilines of the general formula

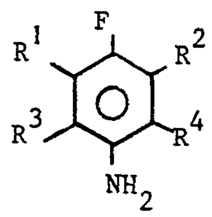

wherein one of $R^1$, $R^2$, $R^3$ and $R^4$ is a hydrogen atom, a halogen atom, a $C_{1-3}$alkyl group or a $CF_3$ group, the others being independently selected from hydrogen atoms, halogen atoms and $C_{1-3}$ alkyl groups, constitute valuable starting materials for the preparation of pharmaceutical products and agrochemicals. They can be prepared in a fairly simple manner by catalytic hydrogenation in hydrogen fluoride of the corresponding nitro compounds containing no substituent in a position para to the nitro group. In this reaction the nitro group is converted into an amino group and a fluorine atom is inserted para to the amino group. In this way, for example, para-fluoroaniline, para-fluoro-meta-chloroaniline and ortho, para-difluoroaniline can be prepared starting from nitrobenzene, meta-chloronitrobenzene and ortho-fluoronitrobenzene, respectively. A drawback to this technique is that in addition to the formation of the desired para-fluoroaniline, it also involves the formation of considerable quantities of the corresponding aniline in which there has been no fluorine substitution in the para position. Often reaction mixtures are obtained in which the anilines formed consist 10-30%m of anilines in the formation of which there has been no fluorine substitution in the para position. Since usually the by-product has a boiling point very close to that of the main product it is extremely difficult to achieve purification of the desired para-fluoroaniline by distillation. This is a major problem. Thus, at atmospheric pressure, para-fluoroaniline has a boiling point of 187 °C, whereas aniline itself has a boiling point of 184 °C, para-fluoro-meta-chloroaniline has a boiling point of 230 °C, whereas meta-chloroaniline has a boiling point of 230 °C, and ortho, para-difluoroaniline has a boiling point of 170 °C, whereas ortho-fluoroaniline has a boiling point of 174 °C.

This difficulty in separating these pairs of compounds forms a major problem when highly pure para-fluoroanilines are required. The difficulties in separating these compounds by means of distillation are discussed, for example, in publications by Fidler et al. J. Org. Chem. 26 (1961), pp. 4014-4017 and Mulvey et al. Tetrahedron Letters 16 - (1978), pp. 1419-1420.

It has now surprisingly been discovered that the mol. percentage of a para-fluoroaniline in a mixture in which this compound occurs together with a corresponding aniline containing no substituent in the para position can be considerably increased by crystallization from an aqueous solution of a non-oxidizing acid. This method also applies to mixtures of N-alkyl-substituted anilines, i.e. mixtures of anilines containing not an $-NH_2$ group, but an $-NHR^5$ or $NR^5R^6$ group, wherein $R^5$ and $R^6$ represent similar or dissimilar alkyl groups.

According to the present invention therefore there is provided a process for separating a para-fluoro aniline from a corresponding para-unsubstituted aniline <u>characterised by</u> dissolving a starting mixture of <u>a para- fluoro</u> aniline of general formula I

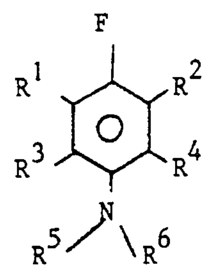

(I)

wherein one of $R^1$, $R^2$, $R^3$ and $R^4$ is a hydrogen atom, a halogen atom, a $C_{1-3}$ alkyl group or a $CF_3$ group, the others being independently selected from hydrogen atoms, halogen atoms and $C_{1-3}$ alkyl groups, and $R^5$ and $R^6$ are selected from hydrogen and alkyl groups, and a corresponding para-unsubstituted aniline of general formula II

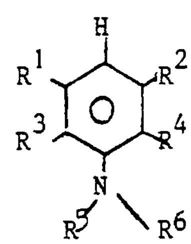

(II)

wherein $R^1$, $R^2$, $R^3$, $R^4$, $R^5$ and $R^6$ are the same as in formula I in an aqueous solution of a non-oxidising acid containing 1 to 5 equivalents of acid per mol of the aniline mixture, and cooling the solution to allow a mixture of salts of the anilines of formulae I and II having a higher mol percentage of the aniline of formula I than in the starting mixture to crystallise out.

Examples of mixtures of anilines that can be subjected to the process according to the invention are:

1) a mixture of para-fluoroaniline and aniline;
2) a mixture of meta-chloro para-fluoroaniline and meta-chloroaniline;
3) a mixture of meta,para-difluoroaniline and meta-fluoroaniline;
4) a mixture of meta-methyl para-fluoroaniline and meta-methylaniline;
5) a mixture of ortho,para-difluoroaniline and ortho-fluoroaniline
6) a mixture of meta-(trifluoromethyl) para-fluoroaniline and meta-(trifluoromethyl)aniline;
7) a mixture of meta-bromo para-fluoroaniline and meta-bromoaniline;
8) a mixture of meta-(isopropyl) para-fluoroaniline and meta-(isopropyl)aniline;
9) a mixture of ortho,meta-dichloro para-fluoroaniline and ortho,meta-dichloroaniline;
10) a mixture of meta$^1$-dichloro para-fluoroaniline and meta, meta$^1$-dichloroaniline;
11) a mixture of meta-chloro meta$^1$-methyl para-fluoroaniline and meta-chloro meta$^1$-methylaniline; and
12) a mixture of ortho,ortho$^1$,meta,meta$^1$-tetrachloro para-fluoroaniline and ortho,ortho$^1$,meta,meta$^1$- tetrachloroaniline.

It is preferred to carry out the process according to the invention using mixtures of anilines having a general formula in which at least two of the groups $R^1$, $R^2$, $R^3$ and $R^4$ are hydrogen atoms. If one or more of the groups $R^1$, $R^2$, $R^3$ and $R^4$ represent alkyl groups, they are preferably methyl groups. If one or more of the groups $R^1$, $R^2$, $R^3$ and $R^4$ represent halogen atoms, they are preferably fluorine and/or chlorine atoms. Preferably $R^5$ and $R^6$ are selected from hydrogen atoms and $C_{1-3}$ alkyl groups. It is further preferred that at least one of the groups $R^5$ and $R^6$ is a hydrogen atom. The process according to the invention is particularly advantageous for increasing the mol percentage of para-fluoroaniline in a mixture containing said compound together with aniline, as well as for increasing the mol percentage of meta-chloro para-fluoroaniline in a mixture containing said compound together with meta-chloroaniline (i.e. where $R^3$ in the formulae I and II is a hydrogen or chlorine atom and $R^1$, $R^2$, $R^4$ and $R^5$ and $R^6$ are all hydrogen atoms).

As observed hereinbefore, mixtures of compounds of the general formulae I and II wherein $R^5$ and $R^6$ represent hydrogen atoms, are obtained in the catalytic hydrogenation in hydrogen fluoride of a compound of general formula II containing a nitro group in the position of the amine group. Compounds wherein at least one of $R^5$ and $R^6$ is an alkyl group may be obtained by alkylation of such mixtures in known manner. Said catalytic hydrogenation is preferably carried out using molecular hydrogen at a temperature of 0 to 150 °C and a pressure of up to 70 × $10^5$ Pa in the presence of 10 to 50 mol hydrogen fluoride per mol of the nitro compound and a hydrogenation catalyst chosen from the group formed by platinum oxide, palladium oxide and palladium metal. Special preference is given to carrying out the catalytic hydrogenation at a temperature of 40 to 75 °C and in the presence of a quantity of 0.05 to 5%w of platinum oxide, palladium oxide or palladium metal, calculated on the quantity by weight of the nitro compound. The process according to the invention is preferably carried out using mixtures of anilines obtained according to the catalytic hydrogenation described hereinabove. As regards the ratio in which the compounds of general formulae I and II are present in the mixture, it is preferred to carry out the process according to the invention using mixtures containing more than 50%m, and more preferably more than 65%m, of the compound of general formula I.

In the process according to the invention the mixture of anilines is conveniently dissolved at an elevated temperature in an aqueous solution of a non-oxidizing acid, and subsequently the solution is cooled, thus allowed a mixture of the salts with an increased mol percentage of the para-fluoroaniline salt to crystallize out. The temperature range wherewithin the dissolution of the aniline mixture and the cooling of the solution take effect is limited in principle at the top by the boiling point of the solution and at the bottom by the solidifying point of the solution. The upper temperature should be chosen at least high enough to allow the mixture of anilines to dissolve completely. Naturally, this temperature will be dependent on the solubility of the anilines in the aqueous solution as well as on their concentration therein. The temperature range over which the solution is cooled to give the desired crystallisation preferably covers at least 40 °C. Preferably, the solution is cooled down to a minimum temperature lying at about ambient temperature. Further, the cooling of the solution is preferably carried out with stirring and at a cooling rate of less than 20 °C per hour.

In the process according to the invention a crystallized product is obtained in which the mol percentage of para-fluoro compound is higher than in the initial mixture. In addition to this purification of the para-fluoro compound there will also be a loss of para-fluoro compound, owing to the fact that part of this compound finds its way into the mother liquor. In the process according to the invention it is important that there should be both a high degree of purification of the para-fluoro compound and no more than a minor loss of the compound. Unfortunately, one is faced here with two conflicting influences, viz. the higher the degree of purity of the para-fluoro compound one aims at, the larger a

loss of that compound one must accept and conversely, a higher level of recovery of the para-fluoro compound will be attended with a lower degree of purity of the compound. The effect of the process according to the invention as regards purification and loss of para-fluoro compound can be influenced to a considerable extent by the degree of concentration chosen for the aniline mixture in the aqueous acid solution and the acidity of the aqueous acid solution. Generally a lower concentration of the aniline mixture in the aqueous acid solution and/or a lower acidity of the aqueous acid solution will lead to a higher degree of purity but a bigger loss of para-fluoro compound, whereas a higher concentration and/or a higher acidity lead to a smaller loss but a lower degree of purity. As regards the concentration of the aniline mixture in the aqueous acid solution, in the process according to the invention, a preferred concentration is 1 to 5mol more preferably 1.5 to 3.5mol, of the aniline mixture per litre of the aqueous acid solution. As regards the acidity, it is preferred to use 1.1 to 2.5 equivalents of acid per mol of the aniline mixture. The process according to the invention is preferably carried out under such conditions as to yield a crystallized product in which the quantity of compound containing no substituent in the para position is less than 50%m of the quantity of this compound present in the initial mixture, whilst in addition as much of the para-fluoro compound present in the initial mixture as possible goes into the crystallized product.

In the process according to the invention use is made of an aqueous solution of non-oxidizing acid. Oxidising acids such as nitric acid and perchloric acid are not eligible. Examples of suitable acids are hydrochloric acid, sulphuric acid, hydrofluoric acid and oxalic acid. An aqueous solution of hydrochloric acid is preferred.

Starting from a mixture with a certain mol percentage of para-fluoro compound, the process according to the invention affords a crystallized product having a higher mol percentage of that compound. Thus a purification of the para-fluoro compound occurs. For instance, starting from a mixture of para-fluoroaniline and aniline with a mol percentage of 70% para-fluoroaniline, a crystallized product could be obtained in which the mol percentage of para-fluoro compound was 90%. According to the desired purity of the para-fluoro compound a single crystallization step may be sufficient in the process according to the invention, or, when further purification of the para-fluoro compound is to be achieved, the crystallized product may be dissolved at an elevated temperature in an aqueous solution of a non-oxidizing acid and cooled to separate therefrom a second crystallized product having an increased mol percentage of para-fluoro compound. In order to enhance the purity of the para-fluoro compound still further, the latter treatment may be repeated once or several times. For instance, in the above-mentioned case in which starting from a mixture of para-fluoroaniline and aniline with a mol percentage of para-fluoroaniline of 70% a crystallized product was obtained which had a mol percentage of para-fluoro compound of 90%, this first crystallized product could be used to prepare a second crystallized product with a mol percentage of para-fluoro compound of 98%, and from this second crystallized product a third crystallized product could be prepared which had a mol percentage of para-fluoro compound of 99.5%.

In those cases where the process according to the invention is used and a higher degree of purity of the para-fluoro compound is required, it is advisable to allow the primarily obtained crystallised product to recrystallize once or several times from an aqueous solution of a non-oxidizing acid. For it has been found that when the desired purity of the para-fluoro compound is thus obtained step-wise, the loss of para-fluoro compound will be smaller than when the same level of purity is realized in a single step.

As observed hereinbefore, the purification of the para-fluoro compound is attended with a loss of the para-fluoro compound, since part of it will go into the mother liquor. Moreover, if in view of the desired high level of purity of the para-fluoro compound the primarily obtained cyrstallized product is recrystallized once or several times from an aqueous solution of a non-oxidizing acid, each recrystallisation step will be attended with a further loss of the para-fluoro compound. For instance, in the above-mentioned case, in which starting from a mixture of para-fluoroaniline and aniline with a mol percentage of para-fluoroaniline of 70% three subsequent crystallization steps resulted in a third crystallized product having a mol percentage of para-fluoro compound of 99.5%, only 34% of the quantity of the para-fluoro compound present in the initial mixture was recovered from the third crystallised product.

The mother-liquor obtained as by-product in the process according to the invention can be evaporated down to produce a mixture of the two compounds whose mol percentage of para-fluoro compound is lower, of course, than that of the mixture that was recrystallised. By a suitable choice of the acidity and concentration when using the process according to the invention the mol percentage of para-fluoro compound in this mixture after evaporation can be increased to a value which is considerably higher than that of the mixture primarily recrystallized.

If in the process according to the invention it is intended to recrystallize the primarily obtained crystallized product once or several times, the loss of para-fluoro compound via the mother liquor can be very suitably reduced by dividing the mixture to be treated into two or more portions of equal composition, subjecting each one of these portions separately to two or more crystallization steps, and adding the mother liquor obtained from crystallization m of portion n to the solution from which crystallization (m-1) of portion (n+1) takes place. For instance, in the above-mentioned case, wherein starting from a mixture of para-fluoroaniline and aniline having a mol percentage of para-fluoroaniline of 70% three subsequent crystallization steps resulted in a third crystallized product having a mol percentage of para-fluoro compound of 99.5%, but wherein only 34% of the quantity of para-fluoro compound present in the initial mixture was recovered from the third crystallized product, the yield of para-fluoro compound of the aforementioned purity could be increased to 43% by carrying out the process as follows. The initial mixture is divided into two portions and each of these portions is subject to three crystallization treatments in which the mother liquor of the second crystallization of the first portion is added to the starting solution for the first crystallization of the second portion whilst the mother liquor of the third crystallization of the first portion is added to the starting solution for the second crystallization of the second portion. Finally, the third crystallized product obtained from the first portion is combined with the third crystallized product obtained from the second portion.

The invention will be further understood from the following illustrative examples.

Example 1

A mixture of para-fluoroaniline and aniline having a mol percentage of para-fluoroaniline of 70% was divided into two portions, viz. portion I consisting of 0.7 mol para-fluoroaniline and 0.3 mol aniline, and portion II consisting of 0.338 mol para-fluoroaniline and 0.145 mol aniline. Portion I was dissolved with stirring at 70 °C in a mixture of 200ml 10N hydrochloric acid and 200ml water. The solution was cooled down to 20 °C with stirring over a period of four hours. The crystallized product obtained was filtered off, and dried to constant weight at 70 °C and 1mm Hg (1.33 × $10^2$Pa). The compositions of the crystallized product (IA1) and the mother liquor (IB1) were determined by means of GLC (gas-liquid chromatography) analysis. The crystallized product IA1 contained 0.406 mol of the hydrochloric acid salt of para-fluoroaniline and 0.046 mol of the hydrochloric acid salt of aniline. Consequently, the mol percentage of para-fluoro compound in the crystallized product IA1 was 90%.

The volume of the mother liquor IB1 was 369ml and it contained 0.294 mol of the hydrochloric acid salt of para-fluoroaniline and 0.254 mol of the hydrochloric acid salt of aniline. Thus, the mol percentage of para-fluoro compound in the mother liquor was 54%.

The crystallized product IA1 which still contained 58% of the para-fluoro compound present in the initial mixture was recrystallized in a way analogous to that of portion I, starting from a mixture of 46.7ml 10N hydrochloric acid and 140.3ml water. Thus were obtained a crystallized product IA2 and a mother liquor IB2. The crystallized product IA2 contained 0.302 mol of the hydrochloric acid salt of para-fluoroaniline and 0.0062 mol of the hydrochloric acid salt of aniline. Consequently, the mol percentage of para-fluoro compound in the crystallized product IA2 was 98%. The volume of the mother liquor IB2 was 187ml and it contained 0.104 mol of the hydrochloric acid salt of para-fluoroaniline and 0.0398 mol of the hydrochloric acid salt of aniline. Consequently, the mol percentage of para-fluoro compound in the mother liquor IB2 was 72%.

The crystallized product IA2 which still contained 43% of the para-fluoro compound present in the initial mixture was recrystallized in a way analogous to that of portion I, starting from a mixture of 32.3ml 10N hydrochloric acid and 96.8ml water. Thus were obtained a crystallized product IA3 and a mother liquor IB3. The crystallized product IA3 contained 0.238 mol of the hydrochloric acid salt of para-fluoroaniline and 0.0011 mol of the hydrochloric acid salt of aniline. Consequently, the mol percentage of para-fluoro compound in the crystallized product IA3 was 99.5%. The volume of the mother liquor IB3 was 129ml and it contained 0.064 mol of the hydrochloric acid salt of para-fluoroaniline and 0.0051 mol of the hydrochloric acid salt of aniline. Consequently, the mol peroentage of para-fluoroaniline in the mother liquor IB3 was 93%. The crystallized product IA3 still contained 34% of the para-fluoro compound present in the initial mixture.

Portion II was recrystallized in a way analogous to that of portion I, starting from a mixture of 16ml 10N hydrochloric acid, 48ml water and mother liquor IB2. Thus were obtained a crystallized product IIA1 and a mother liquor IIB1. The crystallized product IIA1 contained 0.309 mol of the hydrochloric acid salt of para-fluoroaniline and 0.0215 mol of the hydrochloric acid salt of aniline. Consequently, the mol percentage of para-fluoro compound in the crystallized product IIA1 was 93%. The volume of the mother liquor IIB1 was 228.4ml and it contained 0.133 mol of the hydrochloric acid salt of para-fluoroaniline and 0.1633 mol of the hydrochloric acid salt of aniline. Consequently, the mol percentage of parafluoro compound in the mother liquor IIB1 was 45%.

The crystallized product IIA1 was recrystallized in a way analogous to that of portion I, starting from a mixture of 6.8ml 1ON hydrochloric acid, 21ml water and mother liquor IB3. Thus were obtained a crystallized product IIA2 and a mother liquor IIB2. The crystallised product IIA2 contained 0.277 mol of the hydrochloric acid salt of para-fluoroaniline and 0.0085 mol of the hydrochloric acid salt of aniline. Consequently, the mol percentage of para-fluoro compound in the crystallized product IIA2 was 97%. The volume of the mother liquor IIB2 was 156ml and it contained 0.096 mol of the hydrochloric acid salt of para-fluoroaniline and 0.0181 mol of the hydrochloric acid salt of aniline. Consequently, the mol percentage of para-fluoro compound in the mother liquor IIB2 was 84%.

The crystallized product IIA2 was recrystallized in a way analogous to that of portion I, starting from a mixture of 28.5ml 10N hydrochloric acid in 85.5ml water. Thus were obtained a crystallized product IIA3 and a mother liquor IIB3. The crystallized product IIA3 contained 0.205 mol of the hydrochloric acid salt of para-fluoroaniline and 0.001 mol of the hydrochloric acid salt of aniline. Consequently, the mol percentage of para-fluor compound in the crystallized product IIA3 was 99.5%. The volume of the mother liquor IIB3 was 114 mol and it contained 0.072 mol of the hydrochloric acid salt of para-fluoroaniline and 0.0075 mol of the hydrochloric acid salt of aniline. Consequently, the mol percentage of para-fluoro compound in the mother liquor IIB3 was 91%.

Example 2

A mixture of 0.3 mol para-fluoroaniline and 0.2 mol aniline (mol percentage of para-fluoroaniline in the mixture: 60%) was recrystallized in substantially the same manner as portion I of Example 1, starting from a mixture of 83ml 36%w/v hydrochloric acid and 17ml water, with the distinction that in the present instance dissolution took place at 90 C. The crystallized product obtained contained 0.258 mol of the hydrochloric acid salt of para-fluoroaniline and 0.124 mol of the hydrochloric acid salt of aniline. Consequently, the mol percentage of para-fluoro compound in the crystallized product was 67.5%, while the latter still contained 85% of the para-fluoro compound present in the initial mixture. The mother liquor contained 0.042 mol of the hydrochloric acid salt of para-fluoroaniline and 0.076 mol of the hydrochloric acid salt of aniline. Consequently, the mol percentage of para-fluoro compound in the mother liquor was 36%.

Example 3

A mixture of 0.3 mol para-fluoroaniline and 0.2 mol aniline (mol percentage of para-fluoroaniline in the mixture: 60%) was recrystallized in the same manner as described in Example 2, starting from a mixture of 150ml 36%w/v hydrochloric acid and 50ml water. The crystallized product obtained contained 0.225 mol of the hydrochloric acid salt of para-fluoroaniline and 0.045 mol of the hydrochloric acid salt of aniline. Consequently, the mol percentage of para-fluoro compound in the crystallized product was 85%, while the latter still contained 75% of the para-fluoro compound present in the initial mixture. The mother liquor contained 0.075 mol of the hydrochloric acid salt of para-fluoroaniline and 0.155 mol of the hydrochloric acid salt of aniline. Consequently, the mol percentage of para-fluoro compound in the mother liquor was 33%.

Example 4

A mixture of 0.158 mol meta-chloro para-fluoroaniline and 0.067 mol meta-chloroaniline (mol percentage of para-fluoro compound in the mixture: 70%) was recrystallized in the same manner as described in Example 2, starting from a mixture of 50ml 36%w/v hydrochloric acid and 100ml water. The crystallized product obtained contained 0.111 mol of the hydrochloric acid salt of meta-chloro para-fluoroaniline and 0.029 mol of the hydrochloric acid salt of meta-chloroaniline. Consequently, the mol percentage of para-fluoro compound in the crystallized product was 79%, while the latter still contained 70% of the para-fluoro compound present in the initial mixture. The mother liquor contained 0.047 mol of the hydrochloric acid salt of meta-chloro para-fluoroaniline and 0.038 mol of the hydrochloric acid salt of meta-chloroaniline. Consequently, the mol percentage of para-fluoro compound in the mother liquor was 55%.

**Claims**

**1.** A process for separating a para- fluoro aniline from a corresponding para-unsubstituted aniline characterised by dissolving a starting mixture of a para- fluoro aniline of general formula I

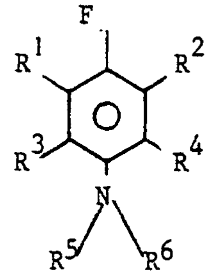

(I)

wherein one of $R^1$, $R^2$, $R^3$ and $R^4$ is a hydrogen atom, a halogen atom, a $C_{1-3}$ alkyl group or a $CF_3$ group, the others being independently selected from hydrogen atoms, halogen atoms and $C_{1-3}$ alkyl groups, and $R^5$ and $R^6$ are selected from hydrogen and alkyl groups, and a corresponding para-unsubstituted aniline of general formula II

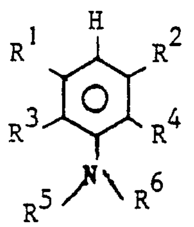

(II)

wherein $R^1$, $R^2$, $R^3$, $R^4$, $R^5$ and $R^6$ are the same as in formula I in an aqueous solution of a non-oxidising acid containing 1 to 5 equivalents of acid per mol of the aniline mixture, and cooling the solution to allow a mixture of salts of the anilines of formulae I and II having a higher mol percentage of the aniline of formula I than in the starting mixture to crystallise out.

2. A process as claimed in claim 1, characterized in that at least two of the groups $R^1$, $R^2$, $R^3$ and $R^4$ are hydrogen atoms.

3. A process as claimed in claim 1 or 2, characterized in that one of $R^1$, $R^2$, $R^3$ and $R^4$ is a hydrogen atom, a fluorine atom, a chlorine atom, a methyl group or a $CF_3$ group, the others being independently selected from hydrogen, fluorine and chlorine atoms and methyl groups.

4. A process as claimed in any one of claims 1 to 3, characterized in that $R^5$ and $R^6$ are selected from hydrogen atoms and $C_{1-3}$ alkyl groups.

5. A process as claimed in any one of claims 1 to 4, characterized in that at least one of $R^5$ and $R^6$ is a hydrogen atom.

6. A process as claimed in any one of claims 1 to 5, characterized in that $R^3$ is a hydrogen atom or a chlorine atom and $R^1$, $R^2$, $R^4$, $R^5$ and $R^6$ are all hydrogen atoms.

7. A process as claimed in any one of claims 1 to 6, characterised in that the aqueous acid solution contains 1.1 to 2.5 equivalents of acid per mol of the aniline mixture.

8. A process as claimed in any one of claims 1 to 7, characterized in that the non-oxidizing acid is hydrochloric acid.

9. A process as claimed in any one of claims 1 to 8, characterized in that the concentration of the aniline mixture in the aqueous acid solution is 1 to 5 mol/1.

10. A process as claimed in claim 9, characterized in that the concentration of the aniline mixture in the aqueous acid solution is 1.5 to 3.5 mol/l.

**Revendications**

1. Un procédé pour séparer une para-fluoro-aniline d'une aniline correspondante non-substituée en position para, caractérisé en ce qu'on dissout un mélange de départ d'une para-fluoro-aniline de formule générale I

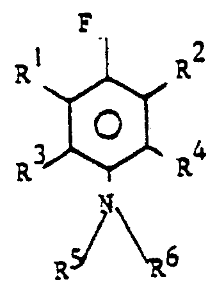

(I)

où un de $R^1$, $R^2$, $R^3$ et $R^4$ est un atome d'hydrogène, un atome d'halogène, un groupe alcoyle en $C_{1-3}$ ou un groupe $CF_3$, les autres étant choisis indépendamment parmi des atomes d'hydrogène, des atomes d'halogènes et des groupes alcoyle en $C_{1-3}$, et $R^5$ et $R^6$ sont choisis parmi l'hydrogène et des groupes alcoyle, et une aniline correspondante non-substituée en position para de formule générale II

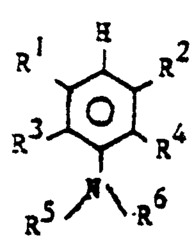

(II)

où $R^1$, $R^2$, $R^3$, $R^4$, $R^5$ et $R^6$ sont les mêmes que dans la formule I dans une solution aqueuse d'un acide non-oxydant contenant 1 à 5 équivalents d'acide par mole du mélange d'anilines, et on refroidit la solution de manière à permettre la cristallisation d'un mélange de sels des anilines des formules I et II ayant un

pourcentage molaire de l'aniline de formule I plus élevé que dans le mélange de départ.

2. Un procédé selon la revendication 1, caractérisé en ce qu'au moins deux des groupes $R^1$, $R^2$, $R^3$ et $R^4$ sont des atomes d'hydrogène.

3. Un procédé selon la revendication 1 ou 2, caractérisé en ce qu'un de $R^1$, $R^2$, $R^3$ et $R^4$ est un atome d'hydrogène, un atome de fluor, un atome de chlore, un groupe méthyle ou un groupe $CF_3$, les autres étant choisis indépendamment parmi des atomes d'hydrogène, de fluor et de chlore et des groupes méthyle.

4. Un procédé selon l'une quelconque des revendications 1 à 3, caractérisé en ce que $R^5$ et $R^6$ sont choisis parmi des atomes d'hydrogène et des groupes alcoyle en $C_{1-3}$.

5. Un procédé selon l'une quelconque des revendications 1 à 4, caractérisé en ce qu'au moins un de $R^5$ et $R^6$ est un atome d'hydrogène.

6. Un procédé selon l'une quelconque des revendications 1 à 5, caractérisé en ce que $R^3$ est un atome d'hydrogène ou un atome de chlore et $R^1$, $R^2$, $R^4$, $R^5$ et $R^6$ sont tous des atomes d'hydrogène.

7. Un procédé selon l'une quelconque des revendications 1 à 6, caractérisé en ce que la solution aqueuse d'acide contient 1,1 à 2,5 équivalents d'acide par mole du mélange d'anilines.

8. Un procédé selon l'une quelconque des revendications 1 à 7, caractérisé en ce que l'acide non-oxydant est de l'acide chlorhydrique.

9. Un procédé selon l'une quelconque des revendications 1 à 8, caractérisé en ce que la concentration du mélange d'anilines dans la solution aqueuse d'acide est de 1 à 5 moles par litre.

10. Un procédé selon la revendication 9, caractérisé en ce que la concentration du mélange d'anilines dans la solution aqueuse d'acide est de 1,5 à 3,5 moles par litre.

## Patentansprüche

1. Verfahren zur Abtrennung eines p-Fluoranilins von einem entsprechenden p-unsubstituierten Anilin, gekennzeichnet durch Lösen eines Ausgangsgemisches aus einem p-Fluoranilin der allgemeinen Formel I

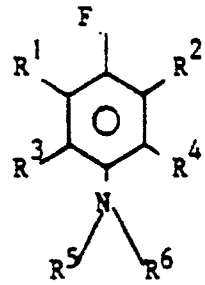

(I)

in der einer der Reste $R^1$, $R^2$, $R^3$ und $R^4$ ein Wasserstoffatom, ein Halogenatom, eine $C_1$- bis $C_3$-Alkylgruppe oder eine $CF_3$-Gruppe ist, und die anderen unabhängig voneinander ausgewählt sind aus Wasserstoffatomen, Halogenatomen und $C_1$- bis $C_3$-Alkylgruppen, und $R^5$ und $R^6$ ausgewählt sind aus Wasserstoff und Alkylgruppen, und einem entsprechenden in p-Stellung unsubstituierten Anilin der allgemeinen Formel II

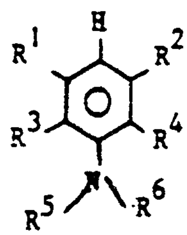

(II)

in der $R^1$, $R^2$, $R^3$, $R^4$, $R^5$ und $R^6$ wie bei Formel I definiert sind, in einer wäßrigen Lösung einer nicht-oxidierenden Säure, enthaltend 1 bis 5 Äq Säure pro mol des Anilingemisches, und Kühlen der Lösung, um ein Gemisch von Salzen der Aniline der Formeln I und II, bei dem der prozentuale Anteil mol des Anilins der Formel I größer ist als in dem Ausgangsgemisch, auszukristallisieren.

2. Verfahren nach Anspruch 1, dadurch gekennzeichnet, daß zumindest zwei der Gruppen $R^1$, $R^2$, $R^3$ und $R^4$ Wasserstoffatome sind.

3. Verfahren nach Anspruch 1 oder 2, dadurch gekennzeichnet, daß einer der Reste $R^1$, $R^2$, $R^3$ und $R^4$ ein Wasserstoffatom, ein Fluoratom, ein Chloratom, eine Methylgruppe oder eine $CF_3$-Gruppe ist und die anderen unabhängig voneinander ausgewählt sind aus Wasserstoff-, Fluor- und Chloratomen und Methylgruppen.

4. Verfahren nach einem der Ansprüche 1 bis 3, dadurch gekennzeichnet, daß $R^5$ und $R^6$ ausgewählt sind aus Wasserstoffatomen und $C_1$- bis $C_3$-Alkylgruppen.

**5.** Verfahren nach einem der Ansprüche 1 bis 4, dadurch gekennzeichnet, daß mindestens einer der Reste $R^5$ und $R^6$ ein Wasserstoffatom ist.

**6.** Verfahren nach einem der Ansprüche 1 bis 5, dadurch gekennzeichnet, daß $R^3$ ein Wasserstoffatom oder ein Chloratom ist und $R^1$, $R^2$, $R^4$, $R^5$ und $R^6$ alles Wasserstoffatome sind.

**7.** Verfahren nach einem der Ansprüche 1 bis 6, dadurch gekennzeichnet, daß die wäßrige Säurelösung 1,1 bis 2,5 Äq Säure pro mol Anilingemisch enthält.

**8.** Verfahren nach einem der Ansprüche 1 bis 7, dadurch gekennzeichnet, daß die nicht-oxidierende Säure Salzsäure ist.

**9.** Verfahren nach einem der Ansprüche 1 bis 8, dadurch gekennzeichnet, daß die Konzentration des Anilingemisches in der wäßrigen Säurelösung 1 bis 5 mol/l beträgt.

**10.** Verfahren nach Anspruch 9, dadurch gekennzeichnet, daß die Konzentration des Anilingemisches in der wäßrigen Säurelösung 1,5 bis 3,5 mol/l beträgt.